# EUROPEAN PATENT APPLICATION

(11) **EP 4 847 029 A1**
(43) Date of publication of application: **02.12.2026**
(21) Application number: 25745264.9
(22) Date of filing: 24.01.2025
(51) Int. Cl.: A61K 35/747, A61P 17/00, A61P 29/00, A23L 33/135, A23K 10/18

(54) **USE OF NOVEL STRAIN FOR TREATING INFLAMMATORY SKIN DISEASES**

(30) Priority: 26.01.2024 KR 20240012108
(71) Applicant: Immunobiome Inc., Nam-gu Pohang-si, Gyeongsangbuk-do 37666 (KR); POSTECH Research and Business Development Foundation, Pohang-si, Gyeongsangbuk-do 37673 (KR)
(72) Inventor: IM, Sin-Hyeog, Pohang-si, Gyeongsangbuk-do 37673 (KR); KIM, Seung Won, Pohang-si, Gyeongsangbuk-do 37673 (KR); PARK, Hye Eun, Pohang-si, Gyeongsangbuk-do 37673 (KR); KIM, Inhae, Pohang-si, Gyeongsangbuk-do 37666 (KR); CHO, Kyungjin, Pohang-si, Gyeongsangbuk-do 37666 (KR)
(74) Representative: Cabinet Becker et Associés
(86) International application number: PCT/KR2025/001549
(87) International publication number: WO 2025/159598

(57) **Abstract**

The present disclosure relates to a novel use of *Lactobacillus plantarum* IMB19 (KCTC 14337BP). The *Lactobacillus plantarum* IMB19 (KCTC 14337BP) according to the present disclosure suppresses inflammatory responses, alleviates itching, and regenerates the stratum corneum, and thus exhibits the effects of preventing, treating, and alleviating inflammatory skin diseases.

## Description

### [Technical Field]

The present disclosure relates to a novel use of *Lactobacillus plantarum* IMB19 KCTC 14337BP.

### [Background Art]

The skin functions to protect the organs of the body from stimuli of the external environment, such as changes in air temperature and humidity and UV rays. However, the skin often comes into direct contact with external stimuli such as UV rays and with various pathogens, and the constant physical and chemical stimuli received from the outside degrade skin function. In addition, since the skin is affected by pathological changes such as heredity, inflammation, benign and malignant tumors, hormones, trauma, and degenerative changes, skin diseases may be caused by a variety of factors.

An inflammatory skin disease refers to a skin disease accompanied by inflammatory responses, such as itching (pruritus), edema, erythema, and peeling, which are caused by various irritating factors that trigger a series of inflammatory responses within the skin epithelium. Known inflammatory skin diseases include allergic dermatitis caused by various factors, bite wounds caused by animal or insect bites, dermatophytosis, and acne. Among them, atopic dermatitis has recently been increasing rapidly and has become a serious problem, due to causes such as the westernization of dietary habits, housing environments, increased stress, and the increase of pollutants accompanying industrial development.

Atopic dermatitis is the most common chronic inflammatory skin disease and is accompanied by severe itching and recurrent eczematous lesions. In infancy, atopic dermatitis begins as eczema on the face and on the extensor surfaces of the limbs; during the growth period, it occurs in the form of eczema on the flexural areas behind the arms or knees; and thereafter, lichenification, in which the skin of the flexural areas thickens, appears. Clinically, atopic dermatitis exhibits broad and heterogeneous characteristics, ranging from eczema confined to the hands to erythema affecting more than 90% of the body surface. Because of this clinical heterogeneity, the prevalence of the disease is difficult to accurately determine. However, its lifetime prevalence worldwide has been reported to reach up to 20%. In particular, whereas it was previously known only as a disease exhibiting a prevalence of up to 25% in infancy, recent reports confirm that adults also exhibit a prevalence of 7% to 10%.

Currently, treatments for inflammatory skin diseases, including atopic dermatitis, include antihistamines, corticosteroids for dermatological use, and phototherapy in which UV light is irradiated after oral administration of psoralen. However, these are effective in only some patients, and therapeutic effects do not appear well in most patients. Moreover, corticosteroids and the like are accompanied by various side effects when used over a long period, and phototherapy likewise has side effects such as an increased risk of skin cancer.

Accordingly, as a result of research on methods capable of effectively treating inflammatory skin diseases, including atopic dermatitis, the present inventors confirmed that *Lactobacillus plantarum* IMB19 KCTC 14337BP has excellent effects in suppressing inflammatory responses, alleviating itching, and regenerating the stratum corneum, thereby completing the present invention.

### [Disclosure]

### [Technical Problem]

One object of the present disclosure is to provide a composition for preventing, treating, or ameliorating an inflammatory skin disease, comprising *Lactobacillus plantarum* IMB19 KCTC 14337BP.

### [Technical Solution]

In the following, in order to prevent confusion arising from redundant content, descriptions of redundant content will be omitted. In other words, the description of the invention is not limited only by the following description, but should be construed in accordance with the overall description of the invention.

Hereinafter, the present disclosure will be described in detail.

The present disclosure provides a composition for preventing, treating, or ameliorating an inflammatory skin disease, comprising *Lactobacillus plantarum* IMB19 KCTC 14337BP.

The *Lactobacillus plantarum* IMB19 of the present disclosure is a lactic acid bacterium isolated and identified from the microorganisms of kimchi.

The 16S rRNA nucleotide sequence for the identification and classification of the *Lactobacillus plantarum* IMB19 of the present disclosure may be one amplified by the forward primer of SEQ ID NO: 1 and the reverse primer of SEQ ID NO: 2 attached to the present specification.
Forward primer (27F primer, SEQ ID NO: 1): AGAGTTTGATCMTGGCTCAG
Reverse primer (1492R primer, SEQ ID NO: 2): TACGGYTACCTTGTTACGACTT

Accordingly, the *Lactobacillus plantarum* IMB19 of the present disclosure may include the 16S rRNA of SEQ ID NO: 3 and SEQ ID NO: 4 as follows.
*Lactobacillus plantarum* IMB19 16S rRNA (785 Forward) (SEQ ID NO: 3): 
AGCGCTGGGATGATGCTAGTGTTGGAGGGTTTCCGCCCTTCAGTGCTGCAGC
TAACGCATTAAGCATTCCGCCTGGGGAGTACGGCCGCAAGGCTGAAACTCAAAGGA
ATTGACGGGGGCCCGCACAAGCGGTGGAGCATGTGGTTTAATTCGAAGCTACGCGA
AGAACCTTACCAGGTCTTGACATACTATGCAAATCTAAGAGATTAGACGTTCCCTTC
GGGGACATGGATACAGGTGGTGCATGGTTGTCGTCAGCTCGTGTCGTGAGATGTTGG
GTTAAGTCCCGCAACGAGCGCAACCCTTATTATCAGTTGCCAGCATTAAGTTGGGCA
CTCTGGTGAGACTGCCGGTGACAAACCGGAGGAAGGTGGGGATGACGTCAAATCAT
 
CATGCCCCTTATGACCTGGGCTACACACGTGCTACAATGGATGGTACAACGAGTTGC
GAACTCGCGAGAGTAAGCTAATCTCTTAAAGCCATTCTCAGTTCGGATTGTAGGCTG
CAACTCGCCTACATGAAGTCGGAATCGCTAGTAATCGCGGATCAGCATGCCGCGGT
GAATACGTTCCCGGGCCTTGTACACACCGCCCGTCACACCATGAGAGTTTGTAACAC
CCAAAGTCGGTGGGGTAACCTTTTAGGAACCAGCCGCCTAAGGTGGGACAGATGAT
TAGGGTGAAGTCGTAACAGGGTAAAACCGTAAAGATGTTCAACCCGCCACATCTGT
CGCGTCTCCGTCGTAGATATAAGAAAGCCAAAGGGCCTTTCTTCCATGGCTGGGTGT
TCATGCAATAACATCGACCGGTTATCCACGACACAAGAAAGGATTACGTTGGTCCTG
GTTGTGCGCTCAGGTTTTATAGTGACAGCGGGCCTATTTGTATGGTGTAAACCGGAG
TGCTAACAATCTTCTACAAGAAACAGCCTGTACATAAATTTACGGCATATATATACC
GGAACGTGGCTTGGCCACGTATGTTATTAACGCGGGCTGGCAGGAACTTACTAGGC
CGTGCCATTCCGGTGTCAAATCCGACCGAATCCGGGGACTCGTCTCGCGGAAATGTG
TTTCTTTTTAGAGACATGGATTCTTACAAACCGAGACCCTGTCATGCCCGGGATGAG
GGTCTGCCACTAACAACTTTCCGAACATGATGGGAAGAACCCCCTAACGGGCGCCC
ACCTGGAGGAATTTGGGCCGGGGCACCACCGCCCGAGGTGGGGCGGAAAACCCCCT
CCAGGGGTCCCATCCTCAATTTTTCCGGGGGGGACCCCCCTCCCCCCCAAAATGAGG
GAAAACCCCCGGGGGGGCACCCCCAAAAGAAGGAGAGCCCCCCACCCTCACTCTTC
CCGCCCGGCGTGCGGGGGCGGGTTTTTTTTTCTGTCAAAATAAATTTGTGTTGTTTG
TGTGTTCCTCCCCCCCCCCGCCGCGGGGGCGGGGTTGTACTTTTTTCCCTCTCCATCCC
CCCCCCACCACAAAAGAAAAGGAGGGGACGACACCCACAGTGGGTGTGTTTTT
*Lactobacillus plantarum* IMB19 16S rRNA (907 Reverse) (SEQ ID NO: 4):

TTGACGGGGGGTCTCCAGGCGGAATGCTTAATGCGTTAGCTGCAGCACTGA
AGGGCGGAAACCCCCCAACACTTAGCATTCATCGTTTACGGTATGGACTACCAGGGT
ATCTAATCCTGTTTGCTACCCATACTTTCGAGCCTCAGCGTCAGTTACAGACCAGAC
AGCCGCCTTCGCCACTGGTGTTCTTCCATATATCTACGCATTTCACCGCTACACATGG

AGTTCCACTGTCCTCTTCTGCACTCAAGTTTCCCAGTTTCCGATGCACTTCTTCGGTT
GAGCCGAAGGCTTTCACATCAGACTTAAAAACCGCCTGCGCTCGCTTTACGCCCAA
TAAATCCGGACAACGCTTGCCACCTACGTATTACCGCGGCTGCTGGCACGTAGTTAG
CCGTGGCTTTCTGGTTAAATACCGTCAATACCTGAACAGTTACTCTCAGATATGTTCT
TCTTTAACAACAGAGTTTACGAGCCGAAACCCTTCTTCACTCACGCGGCGTTGCTC
CATCAGACTTTCGTCCATTGTGGAAGATTCCCTACTGCTGCCTCCCGTAGGAGTTTG
GGCCGTGTCTCAGTCCCAATGTGGCCGATTACCCTCTCAGGTCGGCTACGTATCATT
GCCATGGTGAGCCGTTACCCCACCATCTAGCTAATACGCCGCGGACCATCCAAAA
GTGATAGCCGAAGCCATCTTTCAAACTCGGACCATGCGGTCCAAGTTGTTATGCGGT
ATTAGCATCTGTTTCCAGGTGTTATCCCCCGCTTCTGGGCAGGTTTCCCACGTGTTAC
TCACCAGTTCGCCACTCACTCAAATGTAAATCATGATGCAAGCACCAATCAATACCA
GAGTTCGTTCGACTTGCATGTATTAGGCACGCCGCCAGCGTTCGTCCTGACAGAGAG
AAAAAAAAAAAAAAAAAAGGGCCGGGGGATCGGGGGGGGGGGGGGGGGGTG
AGGGGTTGAGGGGGGGGGGGGGGGGGGGGGGGGGGGGGGGGGGGGGGG
GGGGGGGGGGGGGGGGGGGGGGGGGGGGGGGGGGGGGGGGGGGGGGGG
GGGGGGGGGGGGGGGGGGGGGTGTGTGGGGGGGGGGGGTTGTTGTTTTGTTT
GGGGGGGGGTTGTTTTTGTGTGTGTTTTGTTGTTTGTTTGGGGGTGTGTTTTGTTG
TGGGGTGGGGTGTTGGGGGGGTTGGGGGGGGGTGTTGTTTGGGGGGGTGGGGGG
GGGGGTTTTTTGTTGTTGTGTGGTTGTGTGTTGTGTGGTGGGTGGGGGGGTGGTG
TGTGTGTGGGGGTGGGGGGTGTTTGGTGGGGGGGGGTTGTTGTGGGGGGGTGGTG
TTTGTTTTTTGTTTTTTTTTGTGTGTGGGGGGGGGGGTGGGGGGTGGTTTGTGGGGT
GTTGTTTGTGTGTGGTTGGTGGTGGTGTGTGGGGGGGTTGGGGGGGGGGGGTTGTC
TTTTTTGTTGGTGTTGGGTGTTTGTTGGTGTTGGTGTGTGGTGGGGTGGTGTGGTGGG
TGGGTGCTTGTTGTGTGTGTGGTGTGT

In the present disclosure, the strain may be one deposited at the Korea Research Institute of Bioscience and Biotechnology on October 21, 2020, under accession number KCTC 14337BP.

The present disclosure provides a pharmaceutical composition for preventing or treating an inflammatory skin disease, comprising *Lactobacillus plantarum* IMB19 KCTC 14337BP.

In the present disclosure, the term "inflammatory skin disease" refers to a disease in which a series of clinical signs and symptoms, such as itching, edema, erythema, and peeling, are induced by various irritating factors that cause a series of inflammatory responses within the skin epithelium.

Although not limited thereto, the inflammatory skin disease may be one or more selected from the group consisting of atopic dermatitis, allergic dermatitis, psoriasis, seborrheic dermatitis, contact dermatitis, and lupus erythematosus.

Specifically, the inflammatory skin disease may be atopic dermatitis.

In the present disclosure, the term "atopic dermatitis" refers to a chronic inflammatory skin disease characterized by intense pruritus (e.g., severe itching) and by scaly, dry eczematous lesions. Atopic dermatitis is generally classified, according to its time of onset or the subject in whom it develops, into infantile atopic dermatitis, childhood atopic dermatitis, adult atopic dermatitis, and atopic dermatitis in pregnant women, and atopic dermatitis as used herein is defined as encompassing all of these types of atopic dermatitis.

Specifically, atopic dermatitis exhibits symptoms such as dry eczematous skin and papules, and epidermal hyperplasia, epidermal proliferation, and accumulation of lymphocytes and mast cells are observed in lesion samples from patients with atopic dermatitis. A patient with atopic dermatitis may suffer from severe skin pruritus, which induces inflammation of the skin lesions and thereby further aggravates the clinical symptoms.

The "skin pruritus" of the present disclosure is a disease that encompasses itching occurring due to a decrease in antimicrobial capacity and deterioration of barrier function resulting from a decrease in the lipid content of the stratum corneum of the skin, and itching occurring due to external stimuli such as temperature changes, chemical substances, and electrical stimulation. The scope of the disease according to the present disclosure also encompasses skin pruritus.

The composition according to the present disclosure has the effect of ameliorating symptoms of atopic dermatitis, such as skin inflammation and itching of atopic dermatitis.

According to one embodiment of the present disclosure, *Lactobacillus plantarum* IMB19 KCTC 14337BP may suppress inflammatory responses, alleviate itching, and regenerate the stratum corneum.

Specifically, it was confirmed that *Lactobacillus plantarum* IMB19 KCTC 14337BP exhibits preventive and therapeutic effects against inflammatory skin diseases by (i) reducing the serum IgE concentration and the infiltration of innate immune cells (neutrophils and eosinophils) into skin lesions, (ii) inhibiting the production of cytokines (IL-4 and IL-13), (iii) decreasing an itching-related gene (Areg), and (iv) increasing a stratum corneum-related gene (Lor).

In the present disclosure, "immunoglobulin E (IgE)" is an antibody isotype found only in mammals, and is known to induce a strong inflammatory response despite being the isotype present in the smallest amount in serum.

In the present disclosure, a "neutrophil" is a leukocyte that accounts for the largest proportion in mammals and plays an important role in innate immunity, and is subdivided into segmented neutrophils and banded neutrophils.

In the present disclosure, an "eosinophil" is a type of granulocytic leukocyte having eosinophilic granules in its cytoplasm and is a major cell that participates in allergic responses.

In the present disclosure, "interleukin-4 (IL-4)" is a cytokine that induces the differentiation of unactivated helper T cells into T helper type 2 (Th2) cells and functions as a major regulator of humoral and adaptive immunity.

In the present disclosure, "interleukin-13 (IL-13)" is a cytokine secreted by Th2 cells, CD4 cells, natural killer T cells, mast cells, eosinophils, and nucleated cells, and is known to have secondary structural features and biological functions that are similar to those of IL-4.

In the present disclosure, "amphiregulin (Areg)" refers to a protein synthesized as a transmembrane glycoprotein having 252 amino acids, and is associated with a skin phenotype similar to psoriasis.

In the present disclosure, "loricrin (Lor)" is a terminal differentiation structural protein that accounts for more than 70% of the keratinized envelope and contributes to the protective barrier function of the stratum corneum.

The strain according to the present disclosure may be used in various forms, such as the form of live cells, dead cells, a culture, a lysate, or an extract. Even when the strain according to the present disclosure takes any form under any conditions that include the strain, the strain exhibits results at an equivalent level or higher (in particular, when considered with a focus on live bacteria) in terms of the above-mentioned effects.

Live cells refer to bacteria that are alive as they are, and dead cells refer to those obtained by culturing live bacteria and the like under certain conditions and then separating and extracting the effective components by methods such as heat drying, pressurization, and chemical treatment.

A culture refers to a product obtained by culturing lactic acid bacteria in a known liquid medium or solid medium, and is a concept that includes the strain according to the present disclosure. The product may include lactic acid bacteria. The medium may be selected from known liquid media or solid media, and may be, for example, De Man, Rogosa, and Sharpe (MRS) liquid medium, Gifu Anaerobic Medium (GAM) liquid medium, MRS agar medium, GAM agar medium, or blood liver (BL) agar medium, but is not limited thereto.

A lysate refers to a material having a disrupted form, obtained by separating and processing live cells, dead cells, or a culture thereof through mechanical or chemical methods. For example, a disrupted form may be prepared through bead mills, presses, a sonicator, a microfluidizer, enzyme treatment, and the like.

An extract refers to a material obtained by extracting live cells, dead cells, and/or a lysate by a commonly known extraction method, for example, by extraction with a known extraction solvent such as water or a C1 to C4 alcohol (e.g., methanol or ethanol).

In the present disclosure, the composition may include the strain at a concentration of 1 × 10⁸ to 3 × 10⁹ CFU, and preferably at a concentration of 5 × 10⁸ to 3 × 10⁹ CFU, but is not limited thereto.

The pharmaceutical composition according to the present disclosure may be prepared into a pharmaceutical formulation using methods well known in the art so as to provide rapid, sustained, or delayed release of the active ingredient after being administered to a subject. In preparing the formulation, the pharmaceutical composition according to the present disclosure may additionally include a pharmaceutically acceptable carrier within a range that does not inhibit the activity of the compound of the present disclosure.

The pharmaceutically acceptable carrier includes those commonly used, such as lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, and mineral oil, but is not limited thereto. In addition, the pharmaceutical composition of the present disclosure may include diluents or excipients, such as fillers, extenders, binders, wetting agents, disintegrants, and surfactants, as well as other pharmaceutically acceptable additives.

Administration of the pharmaceutical composition according to the present disclosure may be carried out in a pharmaceutically effective amount. Here, the term "pharmaceutically effective amount" refers to an amount sufficient to prevent or treat a disease at a reasonable benefit/risk ratio applicable to medical treatment. The level of the effective amount may be variously selected by a person skilled in the art, depending on factors such as the formulation method, the condition and body weight of the patient, the sex, age, and disease severity of the patient, the drug form, the route and duration of administration, the excretion rate, and the response sensitivity. As recognized by those skilled in the art, the effective amount may vary depending on the route of treatment, the use of excipients, and the possibility of use together with other drugs. However, for a desirable effect, in the case of an oral administration agent, the composition of the present disclosure may generally be administered to an adult at 0.0001 to 100 mg/kg of body weight per day, and preferably at 0.001 to 100 mg/kg per day, but the administration amount does not limit the scope of the present disclosure in any respect.

The pharmaceutical composition according to the present disclosure may be administered to mammals, such as mice, livestock, and humans, through various routes. The livestock may be, for example, a dog, a cat, a rabbit, a squirrel, a hamster, a guinea pig, a pig, a cow, or a chicken, but is not limited thereto.

Specifically, the pharmaceutical composition according to the present disclosure may be administered orally or parenterally (e.g., by application, or by intravenous, subcutaneous, or intraperitoneal injection), but oral administration is preferred. Solid formulations for oral administration may include powders, granules, tablets, capsules, soft capsules, and pills. Liquid formulations for oral use include suspensions, oral solutions, emulsions, syrups, and aerosols. In addition to water and liquid paraffin, which are commonly used simple diluents, various excipients, for example, wetting agents, sweeteners, aromatics, and preservatives, may be included. Formulations for parenteral administration may be formulated and used, according to conventional methods, in the form of external preparations such as sterilized aqueous solutions, solutions, non-aqueous solvents, suspensions, emulsions, eye drops, eye ointments, syrups, suppositories, and aerosols, and in the form of sterile injectable preparations, and preferably, pharmaceutical compositions in the form of creams, gels, patches, sprays, ointments, plasters, lotions, liniments, eye ointments, eye drops, pastes, or cataplasms may be prepared and used, but the formulation is not limited thereto. Formulations for topical administration may be anhydrous or aqueous, according to clinical prescription. As the non-aqueous solvents and suspensions, propylene glycol, polyethylene glycol, vegetable oils such as olive oil, and injectable esters such as ethyl oleate may be used. As the base for suppositories, Witepsol, Macrogol, Tween 61, cacao oil, laurel oil, glycerogelatin, or the like may be used.

The pharmaceutical composition according to the present disclosure may be administered alone as an individual therapeutic agent or in combination with other therapeutic agents, and may be administered sequentially or simultaneously with conventional therapeutic agents. In addition, the pharmaceutical composition of the present disclosure may be administered in a single dose or in multiple doses. Taking all of the above factors into consideration, it is important to administer an amount that can obtain the maximum effect with a minimum amount and without side effects, and such an amount may be readily determined by a person skilled in the art.

The term "subject" of the present disclosure includes an animal or a human whose symptoms can be ameliorated by administration of the pharmaceutical composition according to the present disclosure. By administering the pharmaceutical composition according to the present disclosure to a subject, an inflammatory skin disease can be effectively prevented and treated.

The term "administration" of the present disclosure refers to introducing a prescribed substance into a human or an animal by any appropriate method, and the route of administration of the pharmaceutical composition according to the present disclosure may be oral or parenteral through any general route, as long as the composition can reach the target tissue. In addition, the pharmaceutical composition according to the present disclosure may be administered by any device by which the active ingredient can move to the target cells.

The preferable administration amount of the pharmaceutical composition according to the present disclosure varies depending on the condition and body weight of the patient, the disease severity, the drug form, and the route and duration of administration, and can be appropriately selected by a person skilled in the art.

The present disclosure provides *Lactobacillus plantarum* IMB19 KCTC 14337BP for use in preventing or treating an inflammatory skin disease.

The present disclosure provides a use of *Lactobacillus plantarum* IMB19 KCTC 14337BP in the manufacture of a medicament for treating an inflammatory skin disease.

The present disclosure provides a method for preventing or treating an inflammatory skin disease, comprising administering *Lactobacillus plantarum* IMB19 KCTC 14337BP to a subject in need thereof.

The present disclosure provides a composition comprising *Lactobacillus plantarum* IMB19 KCTC 14337BP.

The present disclosure provides a food composition for preventing or ameliorating an inflammatory skin disease, comprising *Lactobacillus plantarum* IMB19 KCTC 14337BP.

The "*Lactobacillus plantarum* IMB19" and the "inflammatory skin disease" are the same as described above.

Specifically, the lactic acid bacteria included in the food composition according to the present disclosure may be live cells thereof, dead cells thereof, a culture thereof, a lysate thereof, or an extract thereof, but any form of lactic acid bacteria capable of achieving the effect of preventing or ameliorating an inflammatory skin disease may be used without limitation.

There is no particular limitation on the type of the food. Foods to which the lactic acid bacteria may be added include sausages, meats, breads, chocolate, snacks, candies, confectioneries, ramen, pizza, other noodles, gums, dairy products including ice cream, various soups, beverages, tea, drinks, alcoholic beverages, and vitamin supplements. In the case of formulation as a beverage, the liquid components added in addition to the novel lactic acid bacteria are not limited thereto but may, like ordinary beverages, contain various flavoring agents or natural carbohydrates as additional components. The aforementioned natural carbohydrates may be monosaccharides (e.g., glucose and fructose), disaccharides (e.g., maltose and sucrose), and polysaccharides (e.g., common sugars such as dextrin and cyclodextrin), as well as sugar alcohols such as xylitol, sorbitol, and erythritol.

The type of the food may specifically be a health functional food. The health functional food may contain various nutrients, vitamins, minerals (electrolytes), flavoring agents such as synthetic flavoring agents and natural flavoring agents, coloring agents and enhancers (cheese, chocolate, and the like), pectic acid and salts thereof, organic acids, protective colloidal thickeners, pH adjusters, stabilizers, preservatives, glycerin, alcohol, and carbonating agents used in carbonated beverages. These components may be used alone or in combination, and the proportion of such additives is generally selected within the range of 0.001 to 50 parts by weight per total weight of the composition.

The health functional food is a food that emphasizes the bioregulatory function of the food, and is a food to which added value has been imparted, using physical, biochemical, and biotechnological methods, so as to act and be expressed for a specific purpose. The components of such a health functional food are designed and processed so as to sufficiently exert, on the living body, body-regulating functions related to biological defense, regulation of body rhythm, and the prevention of and recovery from disease, and may contain food-acceptable food auxiliary additives, sweeteners, or functional raw materials.

When the *Lactobacillus plantarum* IMB19 of the present disclosure is used as a health functional food (or a health functional beverage additive), the *Lactobacillus plantarum* IMB19 may be added as is, or may be used together with other foods or food ingredients, and may be used appropriately according to a conventional method.

The pharmaceutical composition and/or the food composition according to the present disclosure may also be utilized for veterinary purposes.

The present disclosure provides an animal feed additive comprising *Lactobacillus plantarum* IMB19 KCTC 14337BP.

The animal feed additive may be an additive for ameliorating atopic dermatitis.

The animal feed additive of the present disclosure may be in the form of a dry or liquid preparation, and, in addition to the *Lactobacillus plantarum* IMB19 strain, may further include other non-pathogenic microorganisms.

In addition to the *Lactobacillus plantarum* IMB19, the animal feed additive of the present disclosure may further include one or more enzyme preparations. The enzyme preparation to be added may be in either a dry or a liquid state, and the enzyme preparation that may be used includes a lipolytic enzyme such as lipase, a sugar-producing enzyme, such as a phytase, which decomposes phytic acid to produce phosphate and inositol phosphate; an amylase, which is an enzyme that hydrolyzes the α-1,4-glycoside bonds contained in starch, glycogen, and the like; a phosphatase, which is an enzyme that hydrolyzes organic phosphoric acid esters; a carboxymethylcellulase, which decomposes cellulose; a xylase, which decomposes xylose; a maltase, which hydrolyzes maltose into two molecules of glucose; and an invertase, which hydrolyzes saccharose to produce a glucose-fructose mixture, or the like.

When the *Lactobacillus plantarum* IMB19 strain of the present disclosure is used as an animal feed additive, the feed raw materials that may be used include various grains and soybean protein as well as peanuts, peas, sugar beets, pulp, grain by-products, animal offal meal, and fish meal, and these may be used, whether unprocessed or processed, without limitation. The processing method is not necessarily limited thereto, but, for example, extrusion, a process in which the feed raw material, in a filled state, is compressed under pressure through a certain outlet, whereby proteins are denatured and their availability is increased, may preferably be used. Extrusion has advantages such as denaturing proteins through a heat treatment process and destroying anti-enzyme factors. In addition, in the case of soybean protein, extrusion can improve the digestibility of the protein, inactivate anti-nutritional factors such as trypsin inhibitor, which is one of the protease inhibitors present in soybeans, and increase the digestibility by protease, thereby increasing the nutritional value of the soybean protein.

### [Advantageous Effects]

*Lactobacillus plantarum* IMB19 KCTC 14337BP of the present disclosure suppresses inflammatory responses, alleviates itching, and regenerates the stratum corneum, thereby exhibiting preventing, treating, and ameliorating effects against inflammatory skin diseases.

### [Description of Drawings]

FIG. 1 is a diagram illustrating the experimental design for evaluating the immune efficacy of *Lactobacillus plantarum* IMB19 KCTC 14337BP in an atopic dermatitis prevention and treatment model.
FIG. 2 is a diagram confirming, for each mouse group, (a) ear photographs and (b) changes in ear thickness and final ear thickness, in order to evaluate the alleviating efficacy of *Lactobacillus plantarum* IMB19 KCTC 14337BP against atopic dermatitis (****p < 0.0001 (Student's t-test)).
FIG. 3 is a diagram showing a graph of the serum IgE concentration for each mouse group (*p < 0.05, **p < 0.01, ***p < 0.001 (Student's t-test)).
FIG. 4 is a diagram showing, as a graph, the percentage of eosinophils within the CD45+ CD11b+ Ly6G- population that infiltrated the ears of each mouse group (*p < 0.05, ****p < 0.0001 (Student's t-test)).
FIG. 5 is a diagram showing, as graphs, the percentages of (a) IL-4+ cells within the CD4+ T cell population and (b) IL-13+ cells within the CD4+ T cell population in the ear skin tissue of each mouse group (*p < 0.05, **p < 0.01, ***p < 0.001 (Student's t-test)).
FIG. 6 is a diagram showing, as a graph, the relative gene expression level of Areg in the ear skin tissue of each mouse group (*p < 0.05, **p < 0.01 (Student's t-test)).
FIG. 7 is a diagram showing, as a graph, the relative gene expression level of Lor in the ear skin tissue of each mouse group (*p < 0.05, ***p < 0.001, ****p < 0.0001 (Student's t-test)).
FIG. 8 is a diagram illustrating the experimental design for evaluating the dose-dependent immune efficacy of *Lactobacillus plantarum* IMB19 KCTC 14337BP in an atopic dermatitis prevention model.
FIG. 9 is a diagram confirming (a) ear photographs and (b) changes in ear thickness and final ear thickness, in order to evaluate the dose-dependent alleviating efficacy of *Lactobacillus plantarum* IMB19 KCTC 14337BP against atopic dermatitis (***p < 0.001, ****p < 0.0001 (Student's t-test)).
FIG. 10 is a diagram illustrating the experimental design for evaluating the immune efficacy of live cells or dead cells of *Lactobacillus plantarum* IMB19 KCTC 14337BP in an atopic dermatitis prevention and treatment model.
FIG. 11 is a diagram confirming, in an experiment using live cells or dead cells of *Lactobacillus plantarum* IMB19 KCTC 14337BP, (a) ear photographs and (b) changes in ear thickness and final ear thickness, in order to evaluate the alleviating efficacy against atopic dermatitis (****p < 0.0001 (Student's t-test)).
FIG. 12 is a diagram in which, in an experiment using live cells or dead cells of *Lactobacillus plantarum* IMB19 KCTC 14337BP, hematoxylin and eosin (H&E) staining was performed on the ear tissue of each control group and experimental group and the cross-sections were observed, for the histological analysis of the atopic lesions.
FIG. 13 is a diagram showing, as a graph, in an experiment using live cells or dead cells of *Lactobacillus plantarum* IMB19 KCTC 14337BP, the percentage of eosinophils within the CD45+ CD11b+ Ly6G- population that infiltrated the ears of each mouse group (*p < 0.05, **p < 0.01, ****p < 0.0001 (Student's t-test)).
FIG. 14 is a diagram illustrating the experimental design for evaluating the immune efficacy of *Lactobacillus plantarum* IMB19 KCTC 14337BP in an atopic dermatitis prevention and treatment model using NC/Nga mice.
FIG. 15 is a diagram confirming (a) ear photographs and (b) changes in ear thickness and final ear thickness, in order to evaluate the alleviating efficacy of *Lactobacillus plantarum* IMB19 KCTC 14337BP against atopic dermatitis using NC/Nga mice (***p < 0.001, ****p < 0.0001 (Student's t-test)).
FIG. 16 is a diagram showing, as a graph, the percentage of eosinophils within the CD45+ CD11b+ Ly6G- population that infiltrated the ears of each mouse group using NC/Nga mice (*p < 0.05, **p < 0.01, ****p < 0.0001 (Student's t-test)).
FIG. 17 is a diagram showing, as graphs, the percentages of (a) IL-4+ cells within the CD4+ T cell population and (b) IL-13+ cells within the CD4+ T cell population in the ear skin tissue of each mouse group using NC/Nga mice (*p < 0.05, **p < 0.01, ****p < 0.0001 (Student's t-test)).

### [Best Mode for Carrying Out the Invention]

Hereinafter, preferred examples are presented to aid in understanding the present disclosure. However, the following examples are provided merely to facilitate an easier understanding of the present disclosure, and the description of the present disclosure is not limited thereby.

### Experimental Example 1. Preparation of lactic acid bacteria

### Experimental Example 1-1. Isolation and culture of lactic acid bacteria

Kimchi was homogenized, and the suspension was collected. Thereafter, the kimchi was serially diluted and streaked onto De Man, Rogosa, and Sharpe (MRS) broth and agar, followed by culture at 37 °C for 48 hours to isolate colonies, which were further cultured for various analyses.

The *Lactobacillus plantarum* IMB19 strain was cultured in MRS broth at 37 °C for 24 to 36 hours. For transmission electron microscopy (TEM), the bacteria were cultured, then streaked onto MRS-1.5% agar (Neogen Corp., USA) and cultured for 24 to 30 hours. The bacterial colonies were drop-cast onto a 2000-mesh graphene-coated copper grid. TEM imaging was performed at 80 kV using JEOL1220 and Hitachi HT7700.

For identification, the cell morphology of the selected isolated strain was investigated under a microscope. Genetic characterization was performed using genomic DNA, and 16S rRNA sequence analysis was performed at Macrogen Inc. (Korea).

The 16S rRNA gene was amplified by direct PCR using universal primers: the forward primer (27F primer, SEQ ID NO: 1), 5'-AGAGTTTGATCMTGGCTCAG-3', and the reverse primer (1492R primer, SEQ ID NO: 2), 5'-TACGGYTACCTTGTTACGACTT-3'.

The 16S rRNA sequence information of the *Lactobacillus plantarum* IMB19 strain is as follows.
*Lactobacillus plantarum* IMB19 16S rRNA (785 Forward) (SEQ ID NO: 3): 
AGCGCTGGGATGATGCTAGTGTTGGAGGGTTTCCGCCCTTCAGTGCTGCAGC
TAACGCATTAAGCATTCCGCCTGGGGAGTACGGCCGCAAGGCTGAAACTCAAAGGA
ATTGACGGGGGCCCGCACAAGCGGTGGAGCATGTGGTTTAATTCGAAGCTACGCGA
AGAACCTTACCAGGTCTTGACATACTATGCAAATCTAAGAGATTAGACGTTCCCTTC
GGGGACATGGATACAGGTGGTGCATGGTTGTCGTCAGCTCGTGTCGTGAGATGTTGG
GTTAAGTCCCGCAACGAGCGCAACCCTTATTATCAGTTGCCAGCATTAAGTTGGGCA
CTCTGGTGAGACTGCCGGTGACAAACCGGAGGAAGGTGGGGATGACGTCAAATCAT
CATGCCCCTTATGACCTGGGCTACACACGTGCTACAATGGATGGTACAACGAGTTGC
GAACTCGCGAGAGTAAGCTAATCTCTTAAAGCCATTCTCAGTTCGGATTGTAGGCTG
CAACTCGCCTACATGAAGTCGGAATCGCTAGTAATCGCGGATCAGCATGCCGCGGT
GAATACGTTCCCGGGCCTTGTACACACCGCCCGTCACACCATGAGAGTTTGTAACAC
 
CCAAAGTCGGTGGGGTAACCTTTTAGGAACCAGCCGCCTAAGGTGGGACAGATGAT
TAGGGTGAAGTCGTAACAGGGTAAAACCGTAAAGATGTTCAACCCGCCACATCTGT
CGCGTCTCCGTCGTAGATATAAGAAAGCCAAAGGCCTTTCTTCCATGGCTGGGTGT
TCATGCAATAACATCGACCGGTTATCCACGACACAAGAAAGGATTACGTTGGTCCTG
GTTGTGCGCTCAGGTTTTATAGTGACAGCGGGCCTATTTGTATGGTGTAAACCGGAG
TGCTAACAATCTTCTACAAGAAACAGCCTGTACATAAATTTACGGCATATATATACC
GGAACGTGGCTTGGCCACGTATGTTATTAACGCGGGCTGGCAGGAACTTACTAGGC
CGTGCCATTCCGGTGTCAAATCCGACCGAATCCGGGGACTCGTCTCGCGGAAATGTG
TTTCTTTTTAGAGACATGGATTCTTACAAACCGAGACCCTGTCATGCCCGGGATGAG
GGTCTGCCACTAACAACTTTCCGAACATGATGGGAAGAACCCCCTAACGGCGCCC
ACCTGGAGGAATTTGGGCCGGGGCACCACCGCCCGAGGTGGGGCGGAAAACCCCCT
CCAGGGGTCCCATCCTCAATTTTCCGGGGGGGACCCCCCTCCCCCCCAAAATGAGG
GAAAACCCCCGGGGGGGCACCCCCAAAAGAAGGAGAGCCCCCCACCCTCACTCTTC
CCGCCCGGCGTGCGGGGGCGGGTTTTTTTTCTGTCAAAATAAATTTGTGTTGTTTG
TGTGTTCCTCCCCCCCCCGCCGCGGGGGCGGGGTTGTACTTTTTCCCTCTCCATCCC
CCCCCCACCACAAAAGAAAAGGAGGGGACGACACCCACAGTGGGTGTGTTTTT
*Lactobacillus plantarum* IMB19 16S rRNA (907 Reverse) (SEQ ID NO: 4):

TTGACGGGGGGTCTCCAGGCGGAATGCTTAATGCGTTAGCTGCAGCACTGA
AGGGCGGAAACCCCCCAACACTTAGCATTCATCGTTTACGGTATGGACTACCAGGGT
ATCTAATCCTGTTTGCTACCCATACTTTCGAGCCTCAGCGTCAGTTACAGACCAGAC
AGCCGCCTTCGCCACTGGTGTTCTTCCATATATCTACGCATTTCACCGCTACACATGG
AGTTCCACTGTCCTCTTCTGCACTCAAGTTTCCCAGTTTCCGATGCACTTCTTCGGTT
GAGCCGAAGGCTTTCACATCAGACTTAAAAACCGCCTGCGCTCGCTTTACGCCCAA
TAAATCCGGACAACGCTTGCCACCTACGTATTACCGCGGCTGCTGGCACGTAGTTAG
CCGTGGCTTTCTGGTTAAATACCGTCAATACCTGAACAGTTACTCTCAGATATGTTCT

TCTTTAACAACAGAGTTTTACGAGCCGAAACCCTTCTTCACTCACGCGGCGTTGCTC
CATCAGACTTTCGTCCATTGTGGAAGATTCCCTACTGCTGCCTCCCGTAGGAGTTTG
GGCCGTGTCTCAGTCCCAATGTGGCCGATTACCCTCTCAGGTCGGCTACGTATCATT
GCCATGGTGAGCCGTTACCCCACCATCTAGCTAATACGCCGCGGACCATCCAAAA
GTGATAGCCGAAGCCATCTTTCAAACTCGGACCATGCGGTCCAAGTTGTTATGCGGT
ATTAGCATCTGTTTCCAGGTGTTATCCCCCGCTTCTGGGCAGGTTTCCCACGTGTTAC
TCACCAGTTCGCCACTCACTCAAATGTAAATCATGATGCAAGCACCAATCAATACCA
GAGTTCGTTCGACTTGCATGTATTAGGCACGCCGCCAGCGTTCGTCCTGACAGAGAG
AAAAAAAAAAAAAAAAAAGGGCCGGGGGGATCGGGGGGGGGGGGGGGGGGTG
AGGGGTTGAGGGGGGGGGGGGGGGGGGGGGGGGGGGGGGGGGGGGGGGGG
GGGGGGGGGGGGGGGGGGGGGGGGGGGGGGGGGGGGGGGGGGGGGGGG
GGGGGGGGGGGGGGGGGGGGGGTGTGGGGGGGGGGGGGTTGTTGTTTTGTTT
GGGGGGGGGTTGTTTTTGTGTGTGTTTTGTTGTTTGTTTGGGGGTGTGTTTTGTTG
TGGGGTGGGGTGTTGGGGGGGTTGGGGGGGGGTGTTGTTTGGGGGGGTGGGGGG
GGGGGTTTTTTGTTGTTGTGTGGTTGTGTGTTGTGTGGTGGGTGGGGGGGTGGTG
TGTGTGTGGGGGTGGGGGGTGTTTGGTGGGGGGGGGTTGTTGTGGGGGGGTGGTG
TTTGTTTTTTGTTTTTTTTGTGTGTGGGGGGGGGGGGTGGGGGGTGGTTTGTGGGGT
GTTGTTTGTGTGTGGTTGGTGGTGGTGTGTGGGGGGGTTGGGGGGGGGGGGTTGTC
TTTTTTGTTGGTGTTGGGTGTTTGTTGGTGTTGGTGTGTGGTGGGGTGGTGTGGTGGG
TGGGTGCTTGTTGTGTGTGTGGTGTGT

In the following examples, the *Lactobacillus plantarum* IMB19 strain was used in a live state (live), or dead cells (heat-killed) were prepared and used.

The optimal conditions for preparing the dead cells were confirmed through experiments, and accordingly, in this experiment, dead cells prepared by subjecting live cells to heat treatment at 70 °C for 30 minutes were used.

### Experimental Example 2. Reagents, equipment, glassware, and consumables

The reagents used in the present disclosure are as shown in Table 1 below.

**[Table 1]**

| No. | Reagent name | Manufacturer | Product no. | Lot no. | Purpose of use |
|---|---|---|---|---|---|
| 1 | 2,4-Dinitrochloroben zene | T&I | C0162 | GI6XC | Preparation of sensitization and induction solutions |
| 2 | Acetone | DUKSAN | 507 | E6GBB1 | Preparation of sensitization and induction solutions |
| 3 | Tween 20 | Sigma-Aldrich | P2287-500ML | MKCP1919 | Preparation of house dust mite induction solution |
| 4 | Olive oil | Sigma-Aldrich | O1514-500ML | BCBT7822 | Preparation of sensitization and induction solutions |
| 5 | FBS | Hyclone | SV30207.02 | SF0005003 | Media preparation |
| 6 | MITE, DUST D. FARINAE | GREER | XPB81D3A25 | 364761 | Preparation of house dust mite induction solution |
| 7 | MITE, DUST D. FARINAE | GREER | XPB81D3A25 | 415035 | Preparation of house dust mite induction solution |
| 8 | MEM Non-Essential Amino Acid Solution | WELGENE | LS 005-01 | LS05180301 | Media preparation |
| 9 | 2-Mercaptoethanol | Gibco | 21985-023 | 2392438 | T cell media preparation |
| 10 | 1M HEPES | WELGENE | BB 001-01 | BB01220101 | Media preparation |
| 11 | 0.5M EDTA | T&I | BED-9180 | P220708L-9180 | Fluorescence-activated cell sorting (FACS) buffer preparation |
| 12 | RPMI 1640 | WELGENE | LM 011-01 | LM11231701 | Media preparation |
| 13 | DPBS | WELGENE | LB 001-02 | LB01231902 | Preparation of 0.5% phosphate-buffered saline with Tween (PBST) |
| 14 | Pen Strep | Gibco | 15140-122 | 2051354 | Media preparation |
| 15 | Collagenase from Clostridium histolyticum (Type V) | Sigma-Aldrich | C9263-500MG | 0000120891 | Ear enzyme media preparation |
| 16 | Olumiant Tab. 2 mg | Lilly | 1126960273 00 | D490751 | Preparation of baricitinib solution |

The equipment, glassware, and consumables used in the present disclosure are as shown in Table 2 and Table 3 below.

**[Table 2]**

| No. | Equipment name | Manufacturer | Model name | Serial no. | Equipment no. |
|---|---|---|---|---|---|
| 1 | Dial Caliper | KORI | A-15 | 115-0702 | N/A |

**[Table 3]**

| No. | Product name | Manufacturer | Product no. | Lot no. |
|---|---|---|---|---|
| 1 | ANIMAL FEEDING NEEDLES | CADENCE SCIENCE | 9921 | 04-14-23 |
| 2 | 28mm Syringe Filter | Corning | 431219 | 14317030 |
| 3 | KOVAX-SYRINGE 30mL | Korea Vaccine | 21G-30mL | 3010605 |

### Experimental Example 3. Preparation of mouse models

For atopic dermatitis induction, BALB/c mice, in which Th2 responses readily occur, or NC/Nga mice, in which atopic dermatitis can be spontaneously induced, were used.

In the case of the BALB/c mice, based on oral administration, 5-week-old mice were used for the prevention model and 8-week-old mice were used for the treatment model. One week before oral administration, the mice were brought into the vivarium for stabilization of the microbiota, and their front toes were clipped for marking.

In the case of the NC/Nga mice, based on oral administration, 5-week-old mice were used for the prevention model and 8-week-old mice were used for the treatment model. One week before oral administration, the mice were brought into the vivarium for stabilization of the microbiota, and their front toes were clipped for marking.

### Experimental Example 4. 1.5% 2,4-dinitrochlorobenzene (DNCB) sensitization

### (1) Preparation of 1.5% DNCB Solution

A DNCB solvent (acetone:olive oil = 1:3) was prepared according to the number of mice. DNCB was weighed according to the dose and transferred to a 5 mL tube, dissolved in the DNCB solvent to the appropriate concentration, and vortexed for one minute.

### (2) Preparation of baricitinib

The total dose of baricitinib required for oral administration to the mice during the experimental period was calculated. Baricitinib was dissolved in phosphate-buffered saline (PBS) according to the dose, filtered using a 28 mm syringe filter, and then dispensed into aliquots for a single oral administration (1 mg/kg).

### (3) 1.5% DNCB Sensitization

The mice were divided into oral administration groups for PBS, baricitinib, and the probiotic strain, respectively. The mice were anesthetized, and 1.5% DNCB solution was applied at 10 µL per side of each ear (a total of 40 µL per animal).

### Experimental Example 5. 1.0% DNCB induction

### (1) Preparation of 1.0% DNCB Solution

The total amount of DNCB solvent (acetone:olive oil = 1:3) required for oral administration to the mice during the experimental period was prepared. DNCB was weighed according to the dose and transferred to a 15 mL tube, dissolved in the DNCB solvent to the appropriate concentration, vortexed for one minute, and then dispensed into aliquots of the amount to be applied once to the ears. The remainder was stored in a -20 °C reagent freezer.

### (2) 1.0% DNCB Induction

PBS, baricitinib, and the probiotic strain were orally administered to the mice by group. The mice were anesthetized, and 1.0% DNCB solution was applied at 10 µL per side of each ear (a total of 40 µL per animal).

### Experimental Example 6. House dust mite (HDM) Induction

### (1) Preparation of HDM solution

0.5% PBST was prepared according to the required amount. HDM was dissolved in 0.5% PBST to a concentration of 10 mg/mL based on the dry weight indicated on the HDM bottle, and dispensed into aliquots of the amount to be applied once to the ears. The remainder was stored in a -20 °C reagent freezer.

### (2) HDM Induction

PBS, baricitinib, and the probiotic strain were orally administered to the mice by group. The mice were anesthetized, and HDM solution was applied at 10 µL per side of each ear (a total of 40 µL per animal).

### Experimental Example 7. Mouse sacrifice

### (1) Preparation for mouse sacrifice

Two E-tubes were prepared per mouse so that blood samples could be obtained. For ear preparation, enzyme media and three each of 20 mL FACS buffer (in a 50 mL Erlenmeyer flask) and 30 mL PBS (in a 50 mL tube) were prepared per mouse and stored at 4 °C.

### (2) Ear preparation

The mouse ears were cut according to the shape of the head and stored, until washing, in a 24-well plate containing PBS. Two 100-Φ Petri dishes were prepared per group to provide four container surfaces. PBS was poured into each container, and keratin and contaminants adhering to the ears were removed by shaking with forceps (three to four times). In the last dish, the hair on the ears was removed, and the ears were cut into four to six pieces and placed in 30 mL PBS (in a 50 mL tube). After shaking for 15 seconds, the pieces were filtered through a metal strainer, and the ear pieces were collected and placed in 20 mL FACS buffer (in a 50 mL Erlenmeyer flask) and stirred at 37 °C for 20 minutes.

During the reaction time, ear enzyme media was prepared. 500 µL each of the media was added to vials, which were sealed and stored in a 37 °C incubator. The 20 mL FACS buffer in which the ear pieces had been incubated with rotation was filtered through a metal strainer, transferred to 30 mL PBS (in a 50 mL tube, stored at 4 °C), and washed. Thereafter, the resulting mixture was filtered through a metal strainer, transferred to 30 mL PBS, and washed once more. Finally, the resulting mixture was filtered through a metal strainer, and the ear tissue was transferred to the vials containing the ear enzyme media. The tissue was finely cut with curved scissors, the remaining volume (4.5 mL) was filled, and stirring was performed at 37 °C for 45 minutes.

After the reaction, with the tissue settled, the sample was pipetted at least 10 times using a pipette aid, and then the tissue was filtered through a mesh and transferred to a 15 mL tube. Thereafter, the vial was washed with 3 mL PBS, filtered once more through a mesh, and transferred to a 15 mL tube. After centrifugation at 4 °C and 1500 rpm for five minutes, the sample was washed with 1 mL T cell media per sample. After centrifugation once more at 4 °C and 1500 rpm for five minutes, the sample was used for FACS staining.

### Example 1. Evaluation of the preventive and therapeutic efficacy of IMB001 against atopic dermatitis

### (1) Preparation of the experiment

To evaluate the preventive efficacy of the *Lactobacillus plantarum* IMB19 strain (IMB001) against atopic dermatitis, IMB001 was orally administered at 5 × 10⁸ CFU to BALB/c mice under specific pathogen-free (SPF) conditions, five times a week for ten weeks. Three weeks after administration of the strain, 1.5% DNCB was applied to both ears of the mice to perform sensitization in order to induce atopic dermatitis.

Meanwhile, to evaluate the therapeutic efficacy against atopic dermatitis, IMB001 was orally administered at 5 × 10⁸ CFU to BALB/c mice under SPF conditions, five times a week for seven weeks. Simultaneously with the start of strain administration, 1.5% DNCB was applied to both ears of the mice to perform sensitization in order to induce atopic dermatitis.

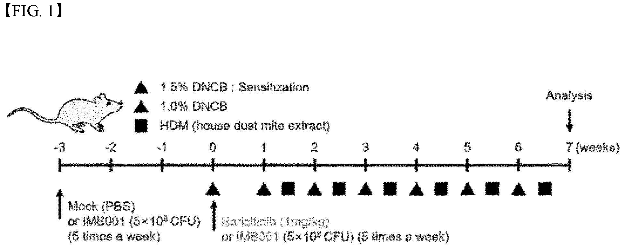

In both conditions, starting one week after sensitization, 1.0% DNCB and HDM were alternately applied to the mouse ears once a week for six weeks, at intervals of three to four days, to induce allergic atopic dermatitis (FIG. 1).

### (2) Measurement of the mouse ear thickness

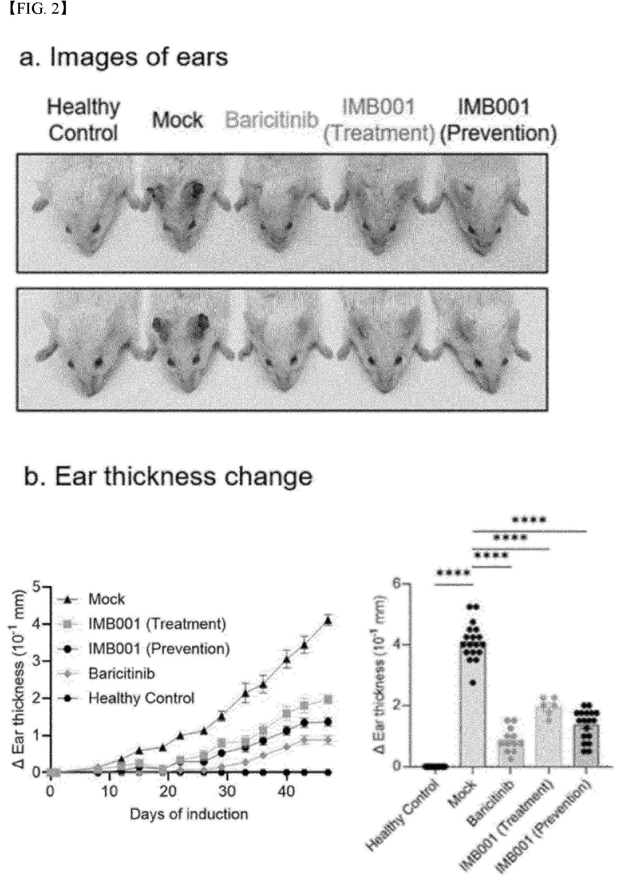

As a result of measuring the mouse ear thickness, the ear thickness of the mock group (negative control) continued to increase until the end of the experiment, whereas the healthy control group exhibited no change in ear thickness. Both the IMB001 prevention group and the IMB001 treatment group exhibited a significantly suppressed increase in ear thickness compared with the mock group (FIGS. 2A and 2B).

### (3) Serum IgE regulatory function

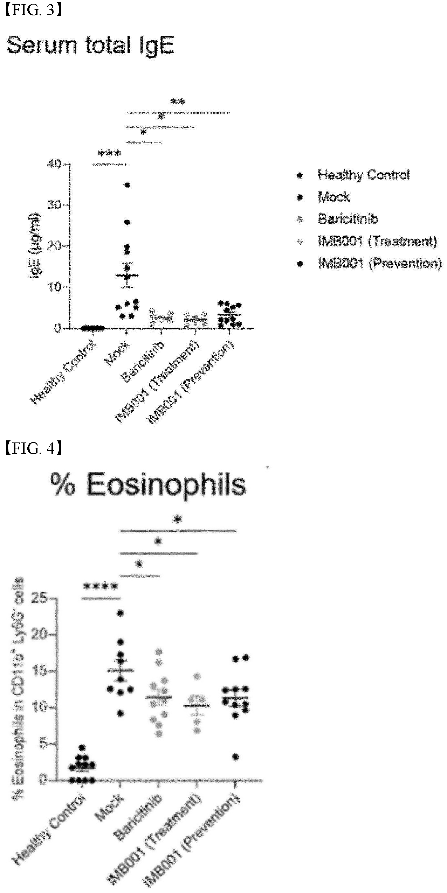

As a result of measuring the serum IgE concentration, which is an important indicator of the symptoms and severity of atopic dermatitis, the serum IgE concentration was significantly reduced in the IMB001 prevention and treatment groups compared with the mock group (FIG. 3).

### (4) Confirmation of the inhibitory efficacy against innate immune cell infiltration

To measure changes in the severity of atopic dermatitis, the innate immune cells that had infiltrated the ear tissue were analyzed by flow cytometry. As a result, in the IMB001 prevention and treatment groups, the percentage of eosinophils within the CD45+ CD11b+ Ly6G- population that had infiltrated the ear tissue was significantly reduced (FIG. 4).

In other words, it was confirmed through the above-described experiments that the symptoms and severity of atopic dermatitis can be alleviated by the administration of *Lactobacillus plantarum* IMB19.

### (5) Evaluation of the cytokine induction and inhibition efficacy

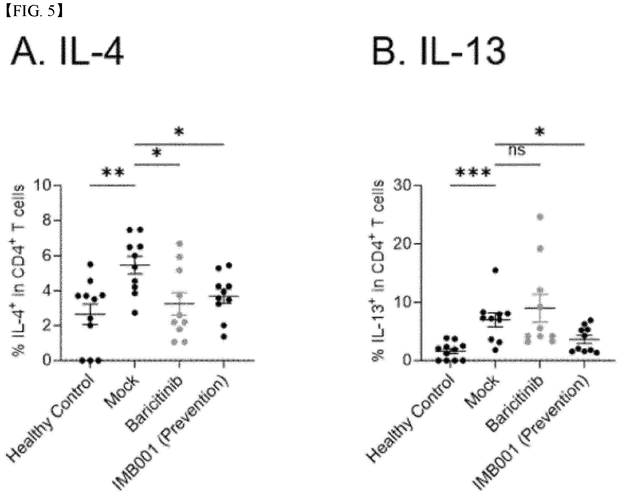

As a result of analyzing the amount of cytokines produced by CD4+ T cells in the ear skin tissue of the mice in each group, IL-4+ CD4+ T cells and IL-13+ CD4+ T cells were significantly reduced in the IMB001 prevention group (FIG. 5).

In other words, it was confirmed that the inflammatory response was suppressed as a result of the reduction of IL-4 and IL-13 induced by atopic dermatitis.

### (6) Analysis of the expression of itching-related genes and stratum corneum-related genes

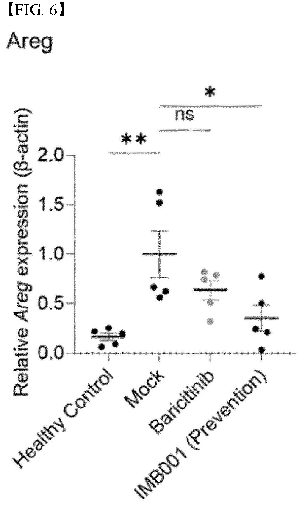

As a result of analyzing the expression of Areg, an inducer associated with the itching symptoms of atopic dermatitis, in the ear skin tissue, a significant decrease was observed in the IMB001 prevention group compared with the mock group (FIG. 6).

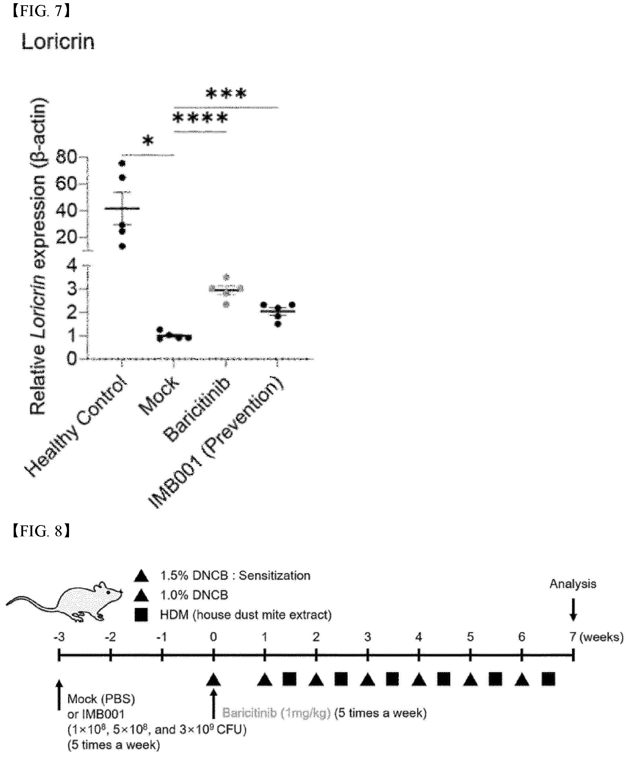

Meanwhile, it was confirmed that the expression level of Lor, which is associated with keratinocyte differentiation and keratinization, was significantly increased in the IMB001 prevention group (FIG. 7).

In other words, it was confirmed that the symptoms of atopic dermatitis were alleviated, as the itching symptoms decreased and the stratum corneum was regenerated.

### Example 2. Evaluation of the preventive efficacy of IMB001 against atopic dermatitis by dose

### (1) Preparation of the experiment

To evaluate the preventive efficacy of the *Lactobacillus plantarum* IMB19 strain (IMB001) against atopic dermatitis by dose, IMB001 was orally administered at a low dose (1 × 10⁸ CFU), an intermediate dose (5 × 10⁸ CFU), and a high dose (3 × 10⁹ CFU) to BALB/c mice under SPF conditions, five times a week for ten weeks. Three weeks after administration of the strain, 1.5% DNCB was applied to both ears of the mice to perform sensitization in order to induce atopic dermatitis.

Starting one week after sensitization, 1.0% DNCB and HDM were alternately applied to the mouse ears once a week for six weeks, at intervals of three to four days, to induce allergic atopic dermatitis (FIG. 8).

### (2) Measurement of the mouse ear thickness

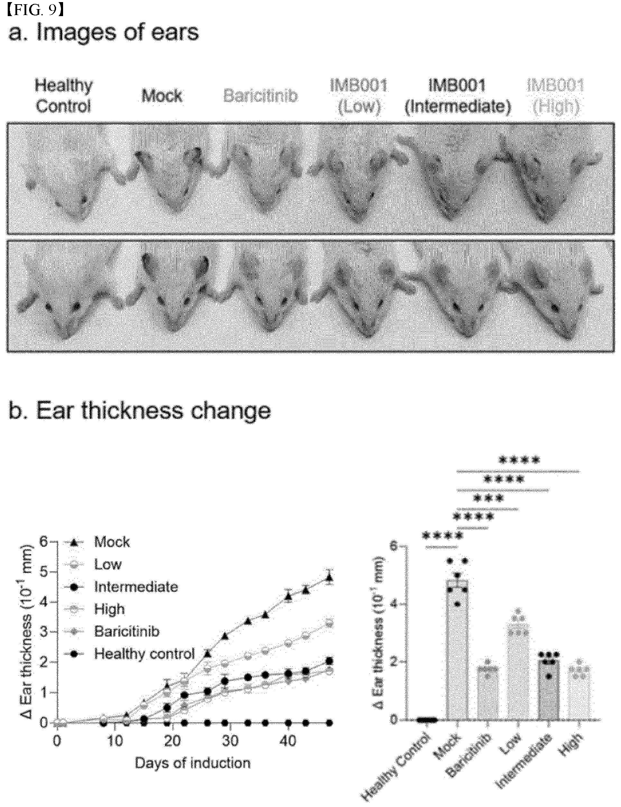

As a result of measuring the mouse ear thickness, the ear thickness of the mock group continued to increase until the end of the experiment, whereas the healthy control group exhibited no change in ear thickness. Compared with the mock group, the increase in ear thickness was significantly suppressed in the low-dose (low) group, the intermediate-dose (intermediate) group, and the high-dose (high) group, and this suppressive effect tended to be more pronounced as the dose increased (FIGS. 9A and 9B).

Through this, it was confirmed that, although an effect on the prevention of atopic dermatitis appears even when only a low dose of *Lactobacillus plantarum* IMB19 is administered, administration at an intermediate dose or a high dose is more effective.

### Example 3. Evaluation of the preventive and therapeutic efficacy of IMB001 dead cells against atopic dermatitis

### (1) Preparation of the experiment

To evaluate the preventive efficacy of live cells and dead cells of the *Lactobacillus plantarum* IMB19 strain (IMB001) against atopic dermatitis, 5 × 10⁸ CFU of live cells were heat-treated at 70 °C for 30 minutes to prepare 5 × 10⁸ CFU of dead cells.

To evaluate the preventive efficacy of live cells and dead cells of the *Lactobacillus plantarum* IMB19 strain (IMB001) against atopic dermatitis, 5 × 10⁸ CFU of IMB001 live cells or 5 × 10⁸ CFU of dead cells were orally administered to BALB/c mice under SPF conditions, five times a week for ten weeks. Three weeks after administration of the strain, 1.5% DNCB was applied to both ears of the mice to perform sensitization in order to induce atopic dermatitis.

Meanwhile, to evaluate the therapeutic efficacy against atopic dermatitis, 5 × 10⁸ CFU of IMB001 live cells or 5 × 10⁸ CFU of dead cells were orally administered to BALB/c mice under SPF conditions, five times a week for seven weeks. Simultaneously with the start of strain administration, 1.5% DNCB was applied to both ears of the mice to perform sensitization in order to induce atopic dermatitis.

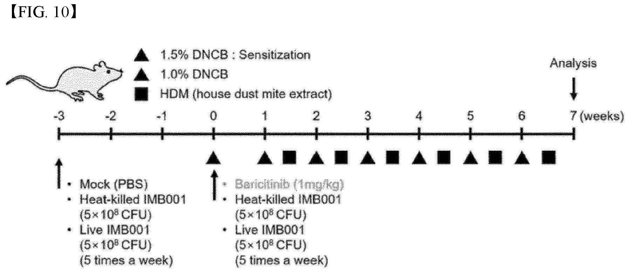

In both conditions, starting one week after sensitization, 1.0% DNCB and HDM were alternately applied to the mouse ears once a week for six weeks, at intervals of three to four days, to induce allergic atopic dermatitis (FIG. 10).

### (2) Measurement of the mouse ear thickness and histological analysis

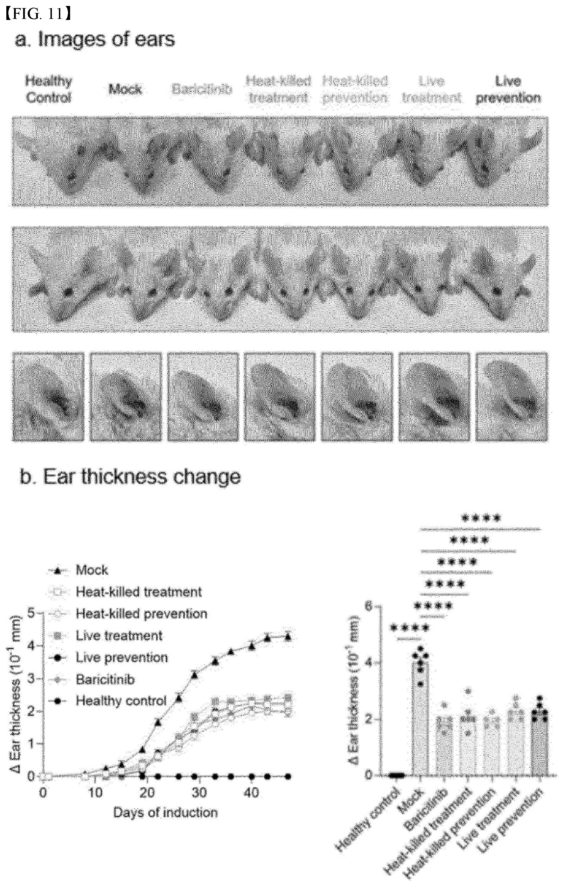

As a result of measuring the mouse ear thickness, the ear thickness of the mock group (negative control) continued to increase until the end of the experiment, whereas the healthy control group exhibited no change in ear thickness. The IMB001 live-cell prevention (live prevention), live-cell treatment (treatment), dead-cell prevention (heat-killed prevention), and dead-cell treatment (heat-killed treatment) groups all exhibited a significantly suppressed increase in ear thickness compared with the mock group (FIGS. 11A and 11B).

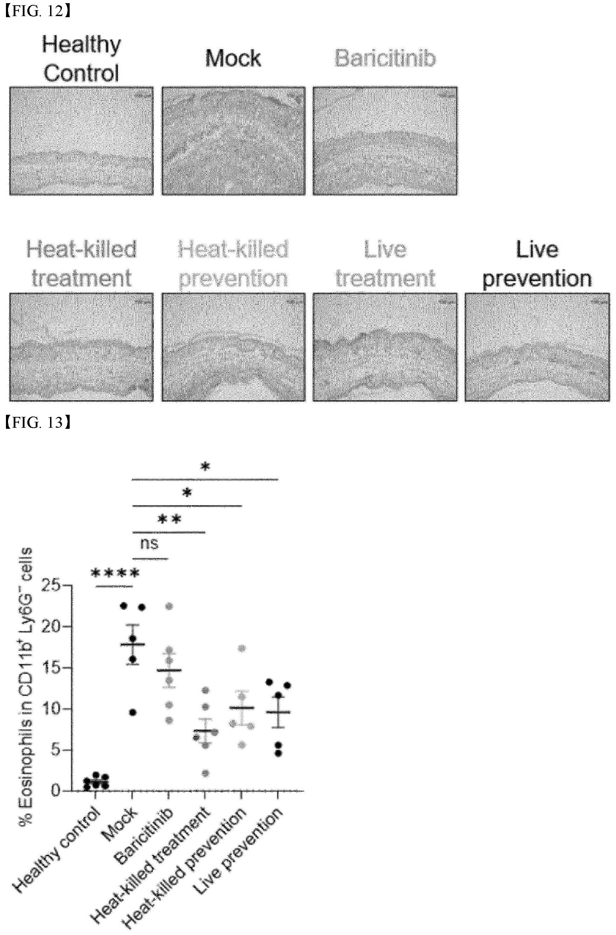

In addition, for the histological analysis of the atopic lesions, hematoxylin and eosin (H&E) staining was performed on the ear tissue of each experimental group at the end of the experiment, and cross-sectional images were obtained (FIG. 12). As a result of image analysis, the tissue thickness of the mock group was the greatest and that of the healthy control group was the smallest, and the IMB001 live-cell prevention (live prevention), live-cell treatment (treatment), dead-cell prevention (heat-killed prevention), and dead-cell treatment (heat-killed treatment) groups all exhibited significantly reduced tissue thickness compared with the mock group.

Through this, it was confirmed that both the live cells and the dead cells of *Lactobacillus plantarum* IMB19 exhibit preventive and therapeutic effects against atopic dermatitis.

### (3) Confirmation of the inhibitory efficacy against innate immune cell infiltration

To measure changes in the severity of atopic dermatitis, the innate immune cells that had infiltrated the ear tissue were analyzed by flow cytometry. As a result, in the IMB001 live-cell prevention (live prevention), dead-cell prevention (heat-killed prevention), and dead-cell treatment (heat-killed treatment) groups, the percentage of eosinophils within the CD45+ CD11b+ Ly6G-population that had infiltrated the ear tissue was significantly reduced (FIG. 13).

Through this, it was confirmed that the live-cell prevention group, or the dead-cell prevention or treatment group, of *Lactobacillus plantarum* IMB19 can alleviate the symptoms and severity of atopic dermatitis.

### Example 4. Evaluation of the preventive and therapeutic efficacy of IMB001 against atopic dermatitis in an NC/Nga mouse model

### (1) Preparation of the experiment

To evaluate the preventive efficacy of the *Lactobacillus plantarum* IMB19 strain (IMB001) against atopic dermatitis, IMB001 was orally administered at 5 × 10⁸ CFU to NC/Nga mice, in which atopic dermatitis can be spontaneously induced, five times a week for eight weeks. Three weeks after administration of the strain, 1.0% DNCB was applied to both ears of the mice to perform sensitization in order to induce atopic dermatitis.

Meanwhile, to evaluate the therapeutic efficacy against atopic dermatitis, IMB001 was orally administered at 5 × 10⁸ CFU to NC/Nga mice, in which atopic dermatitis can be spontaneously induced, five times a week for 5 weeks. Simultaneously with the start of strain administration, 1.0% DNCB was applied to both ears of the mice to perform sensitization in order to induce atopic dermatitis.

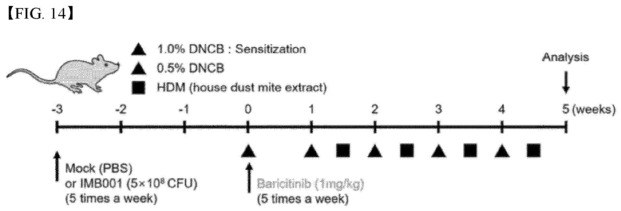

In both conditions, starting one week after sensitization, 0.5% DNCB and HDM were alternately applied to the mouse ears once a week for six weeks, at intervals of three to four days, to induce allergic atopic dermatitis (FIG. 14).

### (2) Measurement of the mouse ear thickness

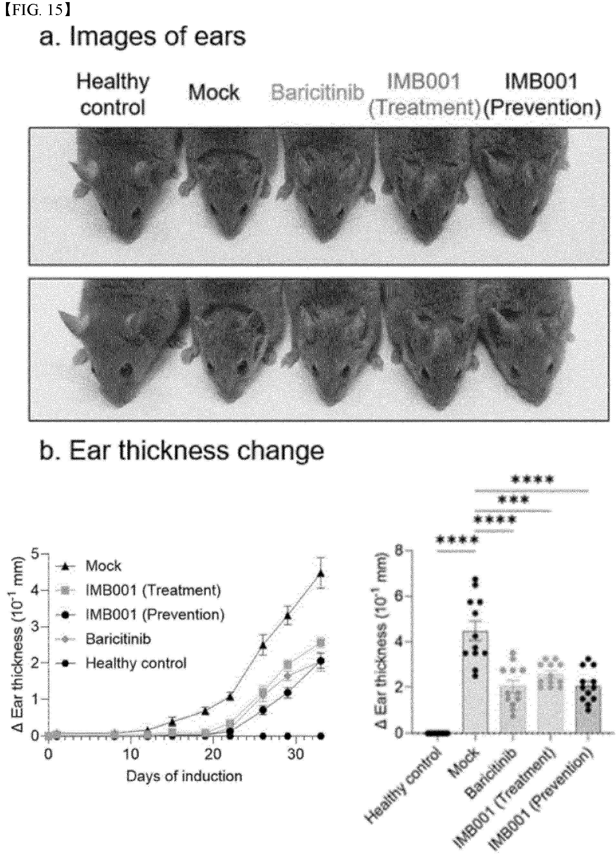

As a result of measuring the mouse ear thickness, the ear thickness of the mock group (negative control) continued to increase until the end of the experiment, whereas the healthy control group exhibited no change in ear thickness. The IMB001 prevention group and the IMB001 treatment group exhibited a significantly suppressed increase in ear thickness compared with the mock group (FIGS. 15A and 15B).

Through this, it was confirmed that *Lactobacillus plantarum* IMB19 has preventive and therapeutic effects against atopic dermatitis in NC/Nga mice.

### (3) Confirmation of the inhibitory efficacy against innate immune cell infiltration

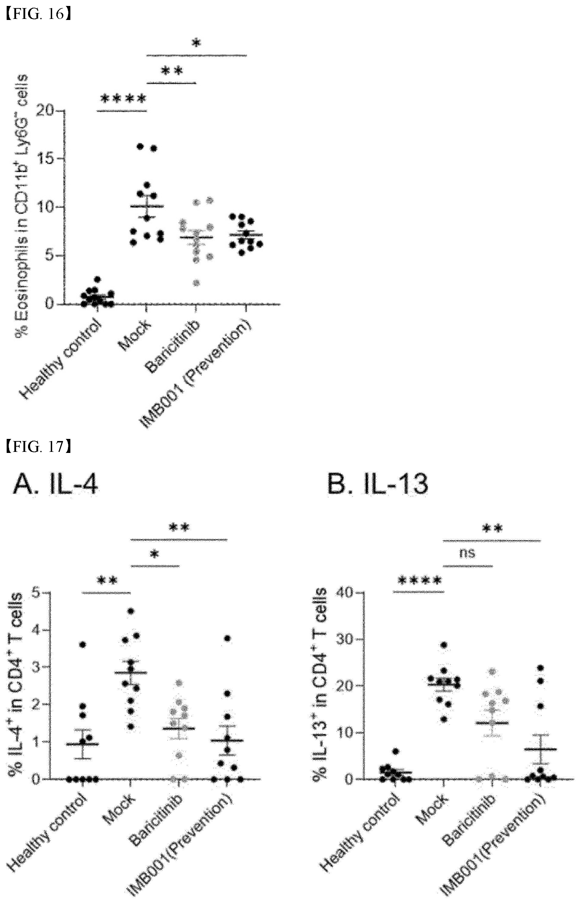

To measure changes in the severity of atopic dermatitis, the innate immune cells that had infiltrated the ear tissue were analyzed by flow cytometry. As a result, in the IMB001 prevention group, the percentage of eosinophils within the CD45+ CD11b+ Ly6G- population that had infiltrated the ear tissue was significantly reduced (FIG. 16).

Through this, it was confirmed that prevention using *Lactobacillus plantarum* IMB19 can alleviate the symptoms and severity of atopic dermatitis in NC/Nga mice.

### (4) Evaluation of the cytokine induction and inhibition efficacy

As a result of analyzing the amount of cytokines produced by CD4+ T cells in the ear skin tissue of the mice in each group, IL-4+ CD4+ T cells and IL-13+ CD4+ T cells were significantly reduced in the IMB001 prevention group (FIG. 17).

Through this, it was confirmed that prevention using *Lactobacillus plantarum* IMB19 suppresses IL-4 induced by atopic dermatitis in NC/Nga mice and also suppresses the inflammatory response.
[Accession Number]
Name of depositary institution: Korea Research Institute of Bioscience and Biotechnology
Accession number: KCTC 14337BP
Date of deposit: October 21, 2020

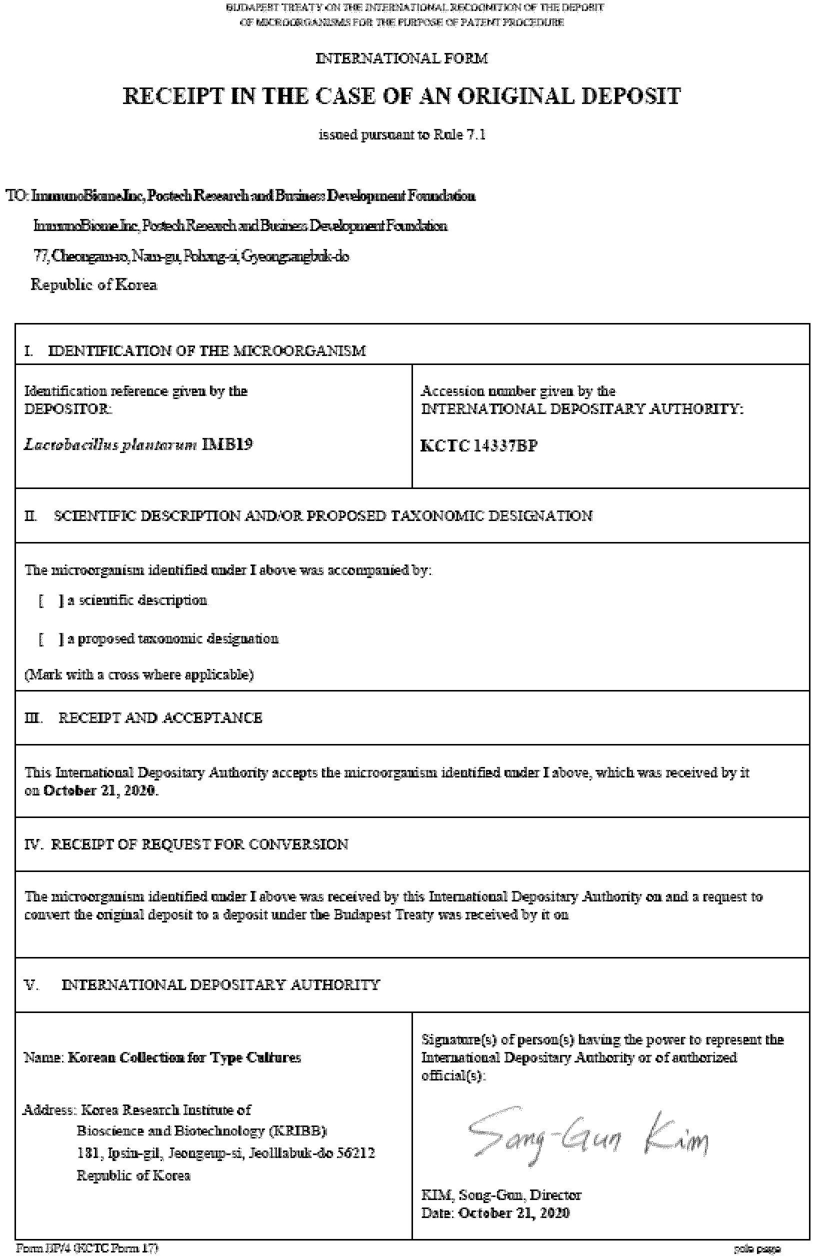
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: ImmunoBiome Inc, Postech Research and Business Development Foundation
ImmunoBiome Inc, Postech Research and Business Development Foundation
77, Cheongam-ro, Nam-gu, Pohang-si, Gyeongsangbuk-do
Republic of Korea

I. IDENTIFICATION OF THE MICROORGANISM
Identification reference given by the
DEPOSITOR:
Lactobacillus plantarum IMB19 | Accession number given by the
INTERNATIONAL DEPOSITARY AUTHORITY:
KCTC 14337BP

II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)

III. RECEIPT AND ACCEPTANCE
This International Depositary Authority accepts the microorganism identified under I above, which was received by it
on October 21, 2020.

IV. RECEIPT OF REQUEST FOR CONVERSION
The microorganism identified under I above was received by this International Depositary Authority on and a request to
convert the original deposit to a deposit under the Budapest Treaty was received by it on

V. INTERNATIONAL DEPOSITARY AUTHORITY
Name: Korean Collection for Type Cultures
Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Song-Gun, Director
Date: October 21, 2020

Form BP/4 (KCTC Form 17) sole page

## Claims

1. A pharmaceutical composition for preventing or treating an inflammatory skin disease, comprising *Lactobacillus plantarum* IMB19 KCTC 14337BP.

2. The pharmaceutical composition of claim 1, wherein the *Lactobacillus plantarum* IMB19 KCTC 14337BP is in the form of live cells, dead cells, a culture, a lysate, or an extract.

3. The pharmaceutical composition of claim 1, wherein the inflammatory skin disease is one or more selected from the group consisting of atopic dermatitis, allergic dermatitis, psoriasis, seborrheic dermatitis, contact dermatitis, and lupus erythematosus.

4. The pharmaceutical composition of claim 3, wherein the inflammatory skin disease is atopic dermatitis.

5. A food composition for preventing or ameliorating an inflammatory skin disease, comprising *Lactobacillus plantarum* IMB19 KCTC 14337BP.

6. The food composition of claim 5, wherein the *Lactobacillus plantarum* IMB19 KCTC 14337BP is in the form of live cells, dead cells, a culture, a lysate, or an extract.

7. The food composition of claim 5, wherein the inflammatory skin disease is one or more selected from the group consisting of atopic dermatitis, allergic dermatitis, psoriasis, seborrheic dermatitis, contact dermatitis, and lupus erythematosus.

8. The food composition of claim 7, wherein the inflammatory skin disease is atopic dermatitis.

9. An animal feed additive for ameliorating atopic dermatitis, comprising *Lactobacillus plantarum* IMB19 KCTC 14337BP.

10. *Lactobacillus plantarum* IMB19 KCTC 14337BP for use in preventing or treating an inflammatory skin disease.

11. A use of *Lactobacillus plantarum* IMB19 KCTC 14337BP in the manufacture of a medicament for treating an inflammatory skin disease.

12. A method for preventing or treating an inflammatory skin disease, comprising administering *Lactobacillus plantarum* IMB19 KCTC 14337BP to a subject in need thereof.
